# EUROPÄISCHE PATENTSCHRIFT

(11) **EP 1 327 414 B1**
(45) Veröffentlichungstag und Bekanntmachung des Hinweises auf die Patenterteilung: **25.02.2009**
(21) Anmeldenummer: 03000389.1
(22) Anmeldetag: 10.01.2003
(51) Int. Cl.: A61B 5/00

(54) **Vorrichtung zur bildgebenden Diagnose von Gewebe**
Device for diagnostic imaging of tissues
Dispositif d'imagerie diagnostique de tissus

(30) Priorität: 11.01.2002 DE 10201005
(43) Veröffentlichungstag der Anmeldung: 16.07.2003
(73) Patentinhaber: Richard Wolf GmbH, 75438 Knittlingen (DE)
(72) Erfinder: Weber, Bernd-Claus, 76228 Karlsruhe (DE); Goll, Thomas, 75438 Knittlingen (DE); Pereira-Delgado, Nicolas, 75433 Maulbronn (DE); Eidner, Phillip, 75015 Bretten-Büchig (DE); Müller, Stefan, 75015 Bretten-Diedelsheim (DE); Schmidt, Olaf, 71665 Vaihingen/Enz (DE); Wagnières, Georges, Dr., 1095 Lutry (CH); van den Bergh, Hubert, Prof. Dr., 1025 St-Stulpice (CH); Glanzmann, Thomas, Dr., 4102 Binningen (CH); Gabrecht, Tanja, 58739 Wickede (DE)
(74) Vertreter: Hemmer, Arnd

(56) Entgegenhaltungen:
- DE-C- 19 902 184
- US-A- 6 110 106

## Beschreibung

Die Erfindung betrifft eine Vorrichtung zur bildgebenden Diagnose von Gewebe unter wahlweiser Anwendung von zwei Diagnosemethoden, nämlich einem Arbeitsmodus zur diagnostischen Weißlicht-Endoskopie (DWLE) und einem Arbeitsmodus zur diagnostischen Autofluoreszenz-Endoskopie (DAFE), mit einer Lichtquelle, deren Licht über ein Endoskop zum Gewebe geleitet wird, und mit einer Bildübertragungseinheit und einer Bildaufnahmeeinheit, welche mit einer Bildverarbeitungseinheit in Verbindung steht, über welche ein Monitor mit einem Bildsignal versorgt wird. Die Bildübertragungseinheit kann auch direkt mit dem menschlichen Auge in Verbindung stehen. In diesem Fall kann auf die Bildaufnahmeeinheit, die Bildverarbeitungseinheit und den Monitor verzichtet werden.

Wird beispielsweise das Gewebe der menschlichen Bronchialschleimhaut mit Strahlung aus dem violetten und blauen Spektralbereich angeregt, fluoresziert dieses Gewebe vor allen Dingen im Grünen und Roten. Beim gesunden Gewebe ist die Fluoreszenz im grünen Spektralbereich stark, wohingegen diejenige im roten Spektralbereich weniger ausgeprägt ist.

Mit zunehmender Gewebeatypie und mit zunehmendem Malignitätsgrad nimmt die Fluoreszenz jedoch ab, wobei der Rückgang in spektraler Hinsicht nicht gleichmäßig stattfindet, sondern wellenlängenabhänig ist: Der Rückgang im Grünen ist stärker ausgeprägt, während die Abschwächung im Roten im Vergleich dazu geringer ist.

Für die Krebsfrüherkennung mittels der DAFE können gemäß diesem Verhalten zwei Effekte für eine Gewebedifferenzierung ausgenutzt werden. Zum einen erscheint atypisches Gewebe dunkler als gesundes Gewebe, und zum anderen erfährt es eine Farbverschiebung vom Grünen zum Roten hin.

Die alleinige Sichtbarmachung und Beobachtung des ersten Effekts, also des Intensitätsrückgangs mit zunehmender Atypie, ist für eine optimale Gewebedifferenzierung nicht ausreichend, da auch gesundes Gewebe unter bestimmten Bedingungen - z. B. wenn die Gewebeoberflächen stark strukturiert und deshalb "lichtschluckend" wirken - dunkel erscheinen kann und deshalb mit (früh-) malignem Gewebe verwechselt werden könnte. Dieser Umstand resultiert in einer verminderten Spezifität.

Eine hohe, nämlich spezifitätssteigernde Bedeutung kommt deshalb dem zweiten Effekt, also der Farbverschiebung vom Grünen zum Roten mit zunehmender Atypie, zu. Wird nämlich neben dem grünen Spektralbereich, in welchem der stärkste Fluoreszenzbeitrag und die ausgeprägtesten Intensitätsänderungen vorliegen, auch der rote Spektralbereich detektiert, dann hebt sich das (früh-) maligne Gewebe durch seine rötlich-braune Farbe von den oben angesprochenen, zum Teil gleichfalls dunkleren, aber gräulich erscheinenden gesunden Gewebepartien ab.

Die Farbverschiebung vom Grünen zum Roten mit zunehmender Gewebeatypie und damit die Farbkontrastierung ist jedoch im Sinne einer guten Gewebedifferenzierung und vor allen Dingen hinsichtlich einer guten Sensitivität für (früh-) maligne Läsionen nicht optimal aufgrund des mit zunehmendem Malignitätsgrad zwar weniger stark ausgeprägten, aber nicht vernachlässigbaren Rückgangs der Rotfluoreszenz. Wäre beispielsweise die Rotfluoreszenz vom Grad der Gewebeatypie unabhängig, also diesbezüglich konstant, wäre eine ausgeprägtere Farbveränderung mit zunehmender Gewebeatypie zu beobachten. Die Eliminierung der gewebezustandsabhängigen Rotfluoreszenz im Bild und die Etablierung eines vom Gewebezustand nahezu unabhängigen und diesbezüglich nahezu konstanten Rotanteils, also einer Rotreferenz, ermöglicht dagegen bei der DAFE eine deutlich verbesserte farbliche Gewebedifferenzierung und damit eine gesteigerte Sensitivität für (früh-) maligne Läsionen, wenn außerdem die Rotreferenz gerade so realisiert wird, dass beim gesunden Gewebe die Grünfluoreszenz die Rotreferenz klar dominiert, und umgekehrt beim kranken Gewebe die Rotreferenz deutlich höher ist als die im Vergleich zum gesunden Gewebe nun klar abgeschwächte Grünfluoreszenz. Die Rotreferenz kann beispielsweise dadurch realisiert werden, dass das zu untersuchende Gewebe während der DAFE neben der Strahlung zur Fluoreszenzanregung zusätzlich mit Strahlung aus dem roten Spektralbereich beaufschlagt wird. Möglichkeiten der Eliminierung der gewebezustandsabhängigen Rotfluoreszenz, welche sich der in erster Näherung gewebezustandsunabhängigen Rotreferenz überlagern würde, sind an anderen Stellen ausführlich beschrieben

Anstatt der Etablierung einer Rotreferenz ist auch die Etablierung einer Farbreferenz denkbar, welche einem anderen Spektralbereich, z. B. dem Blauen, entstammt. Nachfolgend wird jedoch beispielhaft, aber nicht einschränkend der Fall betrachtet, dass die Referenz mit Strahlung aus dem roten Spektralbereich gebildet wird.

Wie schon oben angedeutet wurde, gibt es hinsichtlich einer bestmöglichen Gewebedifferenzierung eine optimale Einstellung für die Rotreferenz gegenüber der Grünfluoreszenz: Der Referenzanteil sollte so hoch bemessen werden, dass beim gesunden Gewebe mit seiner vergleichsweise starken Grünfluoreszenz diese die Rotreferenz deutlich dominiert, so dass das gesunde Gewebe klar grün erscheint. Gleichzeitig sollte atypisches Gewebe, bei welchem die Grünfluoreszenz - bei entsprechender Wahl der Fluoreszenz-Anregungswellenlängen - um einen Faktor fünf bis zehn gegenüber derjenigen beim gesunden Gewebe geringer ist, vom Referenzlicht dominiert sein in der Art, dass dieses Gewebe deutlich rot erscheint. Diese Form der Einstellung der Farbverhältnisse zueinander sorgt durch eine maximale Farbkontrastierung für eine bestmögliche farbliche Gewebedifferenzierung, da so benignes Gewebe auf der einen Seite und (früh-) malignes Gewebe auf der anderen Seite farblich gesehen am weitesten auseinander liegen.

Neben der bestmöglichen farblichen Differenzierungsmöglichkeit verlangt ein weiterer Aspekt, dass idealerweise immer genau diese Einstellung der Farbverhältnisse hergestellt wird: Dem Anwender sollte es möglich sein, bestimmte, sich bei der DAFE darstellende Gewebefarben entsprechenden Graden der Gewebeatypie zuordnen zu können, d. h., dass beispielsweise frühmaligne Läsionen idealerweise immer im annähernd gleichen Rotton erscheinen sollten, welcher sich nach mehrfachem bestimmungsgemäßem Gebrauch des Systems beim Anwender einprägt. Dies resultiert in einem verbesserten Auffinden der verdächtigen Stellen.

Ungünstigerweise wird jedoch bei der oben dargestellten Vorgehensweise die bei der DAFE dem Betrachter vermittelte Gewebefarbe, also im angesprochen Beispiel das Grün-Rot-Verhältnis, nicht nur durch den Gewebezustand beeinflusst, sondern noch durch weitere Faktoren, die nachfolgend genannt werden.

Beispielsweise weist die Fluoreszenz von Patient zu Patient beim gleichen Gewebezustand zum Teil erhebliche Unterschiede auf, wohingegen das Remissionsverhalten der applizierten Referenzstrahlung, also im erläuterten Fall Rotlicht, kaum an die Person gebunden ist. Die Folge ist ein wechselndes, patientenabhängiges Rot-Grün-Verhältnis, so dass sich beispielsweise gesundes Gewebe ohne Ergreifen weiterer Maßnahmen bei unterschiedlichen Patienten in unterschiedlichen Farbtönen darstellt.

Die gleiche Problematik ergibt sich durch absichtlich herbeigeführte oder toleranzbedingte Änderungen am Systemaufbau. Wenn entweder im Beleuchtungs- / Anregungspfad, also auf dem Weg vom Leuchtmittel zum Gewebe, oder im Bildpfad, also auf dem Weg vom Gewebe zum Monitor oder vom Gewebe zum Auge, Komponenten ersetzt werden durch solche mit einer anderen spektralen Transmissionscharakteristik und wenn diese Änderung nicht vernachlässigbar ist, insbesondere nicht homogen, d. h., wenn beispielsweise kein Rückgang oder keine Verstärkung der Transmission um den annähernd gleichen Faktor über den gesamten spektralen Bereich vorliegt, dann führt dies gleichfalls zu einer unerwünscht veränderten Farbdarstellung des Gewebes. Wird beispielsweise im Anregungs- / Beleuchtungspfad ein Lichtleiter mit einer höheren Blautransmission bei unverändert hoher Rottransmission, aber sonst unverändertem Systemaufbau verwendet, dann nimmt auch die induzierte Grünfluoreszenz gegenüber der vom Gewebe remittierten Referenzstrahlung zu, und das gesunde Gewebe beispielsweise erscheint nun in einem gesättigteren Grün, d. h., dass im erläuterten Beispiel, bei welchem die Referenzstrahlung dem Roten entstammte, die Rotanteile nun eine geringere Rolle spielen. Es kann also passieren, dass sich das Gewebe in ganz anderen Farben darstellt. Dieser Umstand verhindert die beliebige Austauschbarkeit von Systemkomponenten und dies insbesondere dann, wenn deren spektrale Transmissionscharakteristik nicht bekannt ist.

Es sind keine bildgebenden Systeme für die DAFE bekannt, bei welchen neben der Autofluoreszenz am Gewebe remittierte Anregungsstrahlung oder remittierte Strahlung aus einem anderen spektralen Bereich als Referenz in die Fluoreszenzbilddarstellung zusätzlich mit einbezogen wird und gleichzeitig versucht wird, die oben dargestellte Problematik der unerwünscht differierenden Farbverhältnisse zu lösen.

Bekannt ist ein System, welches bei der DAFE einen kleinen Anteil der applizierten blauen Strahlung zur Fluoreszenz-Anregung, welcher aber vom Gewebe nicht absorbiert, sondern remittiert wird, als in erster Näherung gewebezustandsunabhängige Referenz gegenüber der gewebezustandsabhängigen Fluoreszenz zusätzlich in die Bilddarstellung aufnimmt. Durch entsprechende Festlegung des relativen Betrags der dem Auge oder der Kamera zugeführten Referenzstrahlung aus dem spektralen Bereich der Strahlung für die Fluoreszenz-Anregung soll erreicht werden, dass beim gesunden Gewebe die Grün- und Rotfluoreszenz die am Gewebe remittierte und detektierte Blaustrahlung dominiert und umgekehrt beim (früh-) malignen Gewebe die daran remittierte und detektierte Blaustrahlung über die gegenüber gesundem Gewebe deutlich abgeschwächte Fluoreszenz dominiert. Gesundes Gewebe erscheint dann grünlich, krankes Gewebe bläulich bis violett.

Die Erzeugung der Blaureferenz im Bild erfolgt durch geringfügige spektrale Überlappung des Transmissionsbereichs eines Fluoreszenz-Anregungsfilters im Strahlengang der Lichtquelle im Anregungspfad mit dem Transmissionsbereich eines Anregungslicht-Blockfilters im Bildpfad. Entscheidend für das Verhältnis zwischen Fluoreszenz und Blaureferenz ist in erster Linie neben dem Gewebezustand und dem untersuchten Patienten selbst, d. h., dessen generellem Gewebe-Fluoreszenzverhalten, das Verhältnis zwischen der spektralen Lage, Breite und Höhe des Transmissionsbereiches des Fluoreszenz-Anregungsfilters einerseits und dem Umfang der Überlappung der Transmissionsbereiche von Anregungsfilter und Blockfilter andererseits. Sind die beiden Filter und insbesondere die die Durchlassbereiche begrenzenden spektralen Flanken erst einmal festgelegt, dann ist eine einfache und schnelle Regulierung des Verhältnisses zwischen Fluoreszenz und Referenz nicht mehr möglich. Weist also beispielsweise ein Patient bei der durch die Filterspezifikation vorgegebenen, bereitgestellten Menge an Anregungsstrahlung eine vergleichsweise geringe, d. h. unterdurchschnittliche Fluoreszenz auf, dann kann die Referenzintensität nicht ohne weiteres dieser geringeren Fluoreszenz angepasst werden, um die hinsichtlich einer bestmöglichen Farbkontrastierung von Gewebe optimale Farbwiedergabe zu erzielen.

Eine ähnliche Problematik ergibt sich, wenn bestimmte Systemkomponenten ausgetauscht werden, was oben bereits erläutert wurde. Problematisch bei diesem System können auch herstellungsbedingte Toleranzen bei den spektralen Flankenlagen des Anregungsfilters in der Lichtquelle und des Blockfilters im Bildpfad sein. Der Bereich der spektralen Überlappung darf nämlich nur sehr klein sein, da der Anteil der detektierten, d. h., der auf dem Bild sichtbaren Referenzstrahlung in der Größenordnung der Fluoreszenzintensität liegen muss und diese sich wiederum nur in der Größenordnung von einem Prozent der Intensität der gesamten remittierten Anregungsstrahlung bewegt. Entsprechend stark machen sich geringfügige, eventuell toleranzbedingte Verschiebungen der Flankenlagen der Filter bemerkbar, und zwar durch Veränderungen in der Farbdarstellung von ein und demselben Gewebezustand.

In der DE 199 02 184 C1 ist eine Möglichkeit aufgezeigt, wie prinzipiell auf einfache Art und Weise der oben dargestellten Problematik begegnet werden könnte. Obwohl es sich dort um medikamenteninduzierte Fluoreszenz und nicht um Autofluoreszenz, also nicht um die Fluoreszenz gewebeeigener Farbstoffe, handelt, wird ein vergleichbares Problem auf recht einfache Weise gelöst. Das spektrale Band, welchem die Strahlung für die Fluoreszenz-Anregung entstammt, liegt dort gleichfalls im Violetten und Blauen, die Fluoreszenz selbst liegt jedoch ausschließlich im roten Spektralbereich. Außerdem fluoresziert nur atypisches Gewebe, d. h., wenn keine verdächtigen Gewebestellen vorliegen, ist die dargestellte Bildszene dunkel. Auch bei diesem System besteht *eine* Aufgabe darin, eine verstellbare Referenz, nämlich ein Blaureferenz zu realisieren, wenngleich auch dieser Referenz eine andere Rolle zukommt als die einer Farbreferenz in der hier vorgestellten Art: Es kann beispielsweise bei völlig gesundem Gewebe und deshalb nicht vorhandener Fluoreszenz eine ausreichend helle Umgebungsbeleuchtung zur besseren Orientierung für den Anwender geschaffen werden. Wird jedoch eine verdächtige Stelle ausfindig gemacht, dann können beispielsweise auch ganz schwach fluoreszierende Randbereiche noch sichtbar gemacht und damit die Ränder und Grenzen der Läsionen festgestellt werden, indem die Blaureferenz auf ein Minimum reduziert wird, so dass sich auch noch eine ganz schwache Rotfluoreszenz davon abhebt und erkannt werden kann.

Die Referenz wird dadurch erzeugt, dass sich - prinzipiell vergleichbar mit dem vorausgehend beschriebenen System - der Transmissionsbereich des Fluoreszenz-Anregungsfilters in der Lichtquelle und der Transmissionsbereich des Anregungslicht-Blockfilters im Bildpfad in einem kleinen spektralen Bereich überlappen. Bei dem Fluoreszenz-Anregungsfilter handelt es sich um ein Interferenzfilter.

Die angestrebte Verstellbarkeit der Referenzintensität wird dadurch realisiert, dass das Fluoreszenz-Anregungsfilter gegenüber der Richtung des Strahlenbündels der Lichtquelle verkippt wird. Mit zunehmendem Kippwinkel verlagert sich die langwellige spektrale Kante des Transmissionsbereiches des Anregungsfilters immer weiter ins Kurzwellige. Dadurch verringert sich der spektrale Überlappungsbereich von Anregungsfilter und Anregungslicht-Blockfilter, und die von der Kamera oder vom Auge detektierte Referenzintensität nimmt ab. Bei dieser Vorgehensweise der Winkelverstellung eines Interferenzfilters zur Manipulation, d. h. wellenlängenselektiven Beeinflussung des Transmissionsbereiches einer Beleuchtungseinrichtung, wird auf ein bekanntes Verfahren zurückgegriffen, welches beispielsweise in der US 5 371 624 beschrieben wurde, auf die auch in der DE 199 02 184 C1 verwiesen wird.

Nachteilig bei der in DE 199 02 184 C1 aufgezeigten Lösung ist allerdings, dass sie ungeeignet ist für ein kombiniertes System für die Fluoreszenzendoskopie und die konventionelle Weißlichtendoskopie, wenn ein schneller und unkomplizierter Wechsel zwischen den beiden Diagnosemethoden möglich sein soll und wenn außerdem das System und insbesondere die Lichtquelle konstruktiv in einfachster Weise und dadurch u. a. kostengünstig und im Hinblick auf die Funktionsfähigkeit zuverlässig aufgebaut sein soll. Dies bedeutet beispielsweise die Einschränkung auf nur ein Leuchtmittel im Lichtprojektor, die Reduktion auf möglichst wenige optische und mechanische Komponenten sowie ein Minimum an mechanisch-konstruktivem Aufwand. Ein schneller Wechsel zwischen den beiden Diagnosemethoden, wie in der vorliegenden Erfindung verlangt, erfordert nämlich ein Ein- und Ausschwenken des Anregungsfilters in und aus dem Strahlengang des Leuchtmittels. Ein kontrolliertes Verkippen desselben Filters aber, wie es bei der Vorgehensweise gemäß der DE 199 02 184 C1 erforderlich ist, und die exakte Beibehaltung des während einer Untersuchung einmal optimierten Kippwinkels ungeachtet des Schwenkvorgangs sind nicht mehr möglich, wenn zusätzlich verlangt wird, dass der mechanische und konstruktive Aufwand und damit auch der Kostenaufwand vergleichsweise gering bleiben sollen. Im Sinne einer konstruktiv einfachen, aber exakt funktionierenden Ausführung und damit im Sinne der Erfindung müssen also dasjenige Bauteil, welches in und aus dem Strahlengang geschwenkt wird, und dasjenige Bauteil, welches im Strahlengang verkippt wird, unterschiedliche Komponenten sein. Letzteres Bauteil soll dementsprechend permanent, d. h. sowohl bei der DAFE als auch bei der DWLE, im Strahlengang bleiben.

Die in der DE 199 02 184 C1 aufgezeigte Vorgehensweise ist damit nur für eine Vorrichtung geeignet, welche ausschließlich für die Fluoreszenzendoskopie vorgesehen ist, keinesfalls jedoch für ein konstruktiv einfaches System mit nur einem Leuchtmittel, welches auch einen vor allen Dingen schnellen und einfachen Wechsel in den Modus der DWLE ermöglichen soll.

Der Erfindung liegt daher die Aufgabe zu Grunde, eine Vorrichtung zur bildgebenden Diagnose von Gewebe, insbesondere für die Untersuchung des Bronchialbereichs, unter wahlweiser Anwendung der diagnostischen Weißlicht-Endoskopie DWLE und der diagnostischen Autofluoreszenz-Endoskopie DAFE zu schaffen, die bei der DAFE durch die Etablierung einer Farbreferenz eine gesteigerte Sensitivität und eine verbesserte Spezifität entsprechend den eingangs gemachten Ausführungen ermöglicht.

Dabei soll die bereitgestellte Referenzstrahlung dosierbar, d. h., deren am Gewebe eintreffende Intensität justierbar gemacht werden, um vor allen Dingen eine Anpassung an die patientenabhängigen Fluoreszenzintensitäten zu ermöglichen. Nur so ist es möglich, gewebezustandsgebundene Verhältnisse der Intensitäten von Fluoreszenz und Referenz, d. h., bestimmte Verhältnisse der Intensitäten von Fluoreszenz und Referenz, welche für den Grad der Gewebeatypie charakteristisch, aber vom Patienten unabhängig sind, und damit gewebezustandsgebundene Farben zu realisieren, welche dann eine optimale optische, nämlich farbliche, Gewebedifferenzierung und dadurch eine ideale Detektion frühmaligner Läsionen erlauben.

Die Möglichkeit der Dosierung der Referenzstrahlung soll es außerdem erlauben, bestimmte Systemkomponenten mit gegebenem spektralem Transmissionsverhalten durch Komponenten mit davon abweichendem spektralem Transmissionsverhalten zu ersetzen: Durch eine Korrektur der Intensität der Referenzstrahlung gelingt es, die für die Gewebedifferenzierung optimalen, an bestimmte Gewebezustände geknüpfte Farbeinstellungen wieder herzustellen. Dadurch ist das System nicht an die ausschließliche Verwendung festgelegter Komponenten mit optischen Elementen mit bereits definiertem spektralem Transmissionsverhalten gebunden. Andere, beispielsweise sich bereits beim Anwender befindliche Komponenten, können verwendet werden, was Flexibilität und außerdem einen Kostenvorteil für den Anwender bedeutet.

Die Dosierung der Referenzstrahlung soll auf einfachste Art und

Weise, d. h., mit geringstem technischem Aufwand für den Systemhersteller einerseits und mit geringstem Aufwand für den Anwender andererseits ausführbar sein.

Von größter Bedeutung dabei ist jedoch, dass ein einfacher und schneller Wechsel, beispielsweise über einen Tastendruck an einer der Systemkomponenten, zwischen der DWLE und der DAFE möglich ist, ohne dass die oben geforderten Manipulationsmöglichkeiten an der Intensität der Referenzstrahlung bei der DAFE irgendeinen wahrnehmbaren Einfluss auf die Bildqualität und insbesondere auf die Farbwiedergabe bei der DWLE hätten.Die Vorrichtung zur Verstellung der Intensität der Referenzstrahlung soll sich außerdem in bzw. an der Lichtquelle befinden, also dort, wo auch die Umschaltung zwischen Bereitstellung von Weißlicht für die DWLE und Bereitstellung von Strahlung für die Fluoreszenz-Anregung und Referenzstrahlung für die DAFE stattfindet. Die Konzentration der mit der Diagnosemoden-Umschaltung zusammenhängenden Systemparameter-Veränderungen auf wenige oder idealerweise eine einzige Systemkomponente, also beispielsweise auf die Lichtquelle, hat den Vorteil, dass fast alle anderen Systemkomponenten durch konventionelle Komponenten ersetzt werden können. Dies kann einen erheblichen Vorteil, insbesondere auch im Hinblick auf die Systemkosten, für den Anwender bedeuten.

Außerdem soll die Lichtquelle nur ein einziges Leuchtmittel benötigen, welches einerseits das Weißlicht für die DWLE liefert, andererseits aber auch die Strahlung für die Fluoreszenz-Anregung und für die Etablierung der Farbreferenz bei der DAFE. Die Anzahl der optischen und mechanischen Komponenten sowie der konstruktive Aufwand sollen auf ein Minimum reduziert werden. Diese Vorgehensweise bedeutet in vielerlei Hinsicht Vorteile, z. B. im Hinblick auf die Größe der Lichtquelle und die Kosten des Systems, aber auch z. B. im Sinne der Einfachheit der technischen Umsetzung und daraus resultierend auch im Sinne einer zuverlässigen Funktionsweise.

Eine diese Aufgabe lösende Vorrichtung ist im Anspruch 1 angegeben. Vorteilhafte Weiterbildungen einer solchen Vorrichtung ergeben sich aus den Unteransprüchen.

Bei der erfindungsgemäßen Vorrichtung emittiert die Lichtquelle im Arbeitsmodus der DAFE neben der Strahlung zur Fluoreszenz-Anregung zusätzlich noch Strahlung aus einem Wellenlängenband, welche als Referenz dient. Die Strahlung für die Fluoreszenz-Anregung wird im Bildpfad, also auf dem Weg vom Gewebe zum Monitor, geblockt, und die Referenzstrahlung wird im Bildpfad zumindest partiell transmittiert. Da die vom Gewebe remittierte Referenzstrahlung die Funktion einer Farbreferenz übernehmen soll und da die Autofluoreszenz von gesundem Gewebe der menschlichen Bronchialschleimhaut integral betrachtet grün erscheint, ist als spektrales Band für die Farbreferenz idealerweise ein Wellenlängenbereich im Blauen oder im Roten in Betracht zu ziehen, um eine gute farbliche Gewebedifferenzierung zu realisieren. Wie schon an anderer Stelle erläutert wurde, ist dann die Intensität der bereitgestellten Referenzstrahlung so zu dosieren, dass beim gesunden Gewebe dessen durch die Anregung erzeugte und detektierte Grünfluoreszenz die Intensität und damit auch die dem Betrachter vermittelte Farbe der am Gewebe remittierten und detektierten Referenzstrahlung, also idealerweise entweder blau oder rot, dominiert. Andererseits soll aber die Intensität der am Gewebe remittierten und detektierten Referenzstrahlung so hoch sein, dass diese Intensität und damit auch die vermittelte Farbe die gegenüber gesundem Gewebe um einen Faktor fünf bis zehn reduzierte Grünfluoreszenz von (früh-) malignem Gewebe dominiert. Gesundes Gewebe erscheint dann grün und krankes Gewebe in der Farbe der Referenzstrahlung.

Um die geforderte einfache Dosierbarkeit der Referenzstrahlung zu realisieren, wird, ähnlich wie es in der DE 199 02 184 C1 beschrieben ist, in den Strahlengang des Leuchtmittels ein Kantenfilter eingebracht, dessen optische Filterwirkung mit dielektrischen Schichten erzielt wird. Dieses Kantenfilter ist dadurch gekennzeichnet, dass es diesseits der spektralen Kante blockt, also eine Transmission aufweist, die gegen Null geht, und jenseits der Kante eine sehr hohe Transmission aufweist, die idealerweise gegen hundert Prozent geht, und dass sich durch Verkippen des Filters gegen das parallele Strahlenbündel des Leuchtmittels die spektrale Lage der Kante verschiebt. Im Gegensatz zur Vorrichtung nach der DE 199 02 184 C1 handelt es sich jedoch hier um ein zusätzliches optisches Filter, welches im Sinne einer mechanisch-konstruktiv einfachen praktischen Umsetzung für die gesamte Dauer einer Systemanwendung, also auch beim Wechsel in den Arbeitsmodus der DWLE, mit seinem genau eingestellten und damit optimierten Kippwinkel im Strahlengang des Leuchtmittels verbleiben soll und welches damit nicht identisch sein kann mit dem Filter für die Fluoreszenz-Anregung - letzteres soll ja bei der vorliegenden Erfindung in einfacher Weise in den Strahlengang ein- und ausgeschwenkt werden können, um einen Wechsel zwischen der DWLE und der DAFE zu ermöglichen - aber auf dieses Fluoreszenz-Anregungsfilter abgestimmt sein muss in der Form, dass sich durch Verkippen des Kantenfilters die spektrale Kante über denjenigen spektralen Transmissionsbereich des Anregungsfilters verschiebt, welcher für die Referenzstrahlung verantwortlich ist, wodurch die Intensität der Referenzstrahlung, mit welcher das Gewebe zusätzlich zur Strahlung für die Fluoreszenz-Anregung beaufschlagt wird, verändert werden kann.

Diese Vorgehensweise bedeutet jedoch, dass bestimmte spektrale Bereiche, welche durch die Spezifikation des Kantenfilters und dessen eingestellten Kippwinkel festgelegt sind, geblockt werden und damit für die Bestrahlung und Beleuchtung des Gewebes nicht zur Verfügung stehen, und zwar sowohl im Modus der DAFE als auch im Modus der DWLE. Soll also im Gegensatz zum System gemäß der DE 199 02 184 C1 das System auch uneingeschränkt für die DWLE tauglich sein und soll das Kantenfilter - wie bereits oben dargestellt - im Sinne eines einfachen Systemaufbaus und eines einfachen und schnellen Wechsels zwischen den Arbeitsmoden der DAFE und der DWLE in seiner bei der DAFE exakt eingestellten und damit für den jeweiligen Patienten optimierten Position im Strahlengang des Leuchtmittels über den gesamten Zeitraum einer Untersuchung fixiert bleiben, also sowohl im Modus der DAFE als auch im Modus der DWLE unbewegt im Strahlengang bleiben, dann fehlen bei der DWLE die durch dieses Filter geblockten spektralen Anteile, die aber für eine naturgetreue Farbwiedergabe des Gewebes im Modus der DWLE notwendig sind. Bei der in der DE 199 02 184 C1 vorgestellten Vorrichtung wäre das der gesamte grüne und rote Spektralbereich, welcher geblockt wird und bei Ausführung einer DWLE fehlen würde.

Deshalb ist die erfindungsgemäße Vorrichtung - im Gegensatz zur DE 199 02 184 C1 - weiterhin dadurch gekennzeichnet, dass erstens derjenige spektrale Transmissionsbereich des Fluoreszenz-Anregungsfilters, welcher die Referenzstrahlung bereitstellen soll, entweder am kurzwelligen oder am langwelligen Ende des sichtbaren Spektralbereichs liegt, dass zweitens dieser Transmissionsbereich so schmal gewählt wird, dass er praktisch keinen merklichen Beitrag zur Farbwiedergabe im Modus der DWLE leisten kann, dass drittens sich die spektrale Kante des zusätzlichen, permanent sich im Strahlengang des Leuchtmittels befindlichen Kantenfilters im möglichen, d. h. konstruktiv vorgegebenen Kippbereich idealerweise ausschließlich über den spektralen Bereich, welcher die Referenzstrahlung bereitstellen soll, bewegt - zumindest nicht weiter in den sichtbaren Spektralbereich hinein - und dass viertens der sichtbare spektrale Bereich im Transmissionsbereich dieses Kantenfilters liegt. Die dritte Forderung stellt sicher, dass auch bei Einstellung des ungünstigsten Kippwinkels im Sinne einer maximalen Blockung des Referenzlichts nur ein ganz minimaler Strahlungsanteil im sichtbaren Spektrum geblockt wird, was entsprechend der zweiten Forderung keine merkliche Einschränkung hinsichtlich der Farbwiedergabe bei der DWLE bedeutet.

Bei dieser Vorgehensweise kommt folgende Tatsache zum Tragen: Die bei Bestrahlung der menschlichen Bronchialschleimhaut am Gewebe remittierte Strahlung, d. h. diejenige Strahlung, welche in ihrer Wellenlänge unverändert reflektiert und zurückgestreut wird und z. B. auch die Grundlage für die DWLE darstellt, ist um zwei Größenordnungen, also etwa um einen Faktor hundert, intensiver als die im gesunden Gewebe erzeugte Fluoreszenzstrahlung, also diejenige Strahlung, welche durch eine Transformation von einfallender Strahlung mit einer bestimmten Wellenlänge in Strahlung mit höherer Wellenlänge im Gewebe entsteht. Da einerseits entsprechend den obigen Ausführungen die Intensität der vom Sensor detektierten Referenzstrahlung in der Größenordnung der Intensität der Gewebe-Fluoreszenzstrahlung liegen soll, es sich aber andererseits bei der Referenzstrahlung um remittierte Strahlung und nicht um Fluoreszenzstrahlung handelt, genügen Intensitäten der Referenzstrahlung im Beleuchtungspfad, die um etwa zwei Größenordnungen geringer sind als die Intensitäten der Fluoreszenz-Anregungsstrahlung. Dabei wird zunächst vereinfachend davon ausgegangen, dass die Fluoreszenzstrahlung und die Referenzstrahlung vom Sensorsystem der Bildaufnahmeeinheit gleich gut detektiert werden. Bei Abweichungen von dieser vereinfachenden Annahme, müssen entsprechende Korrekturfaktoren berücksichtigt werden.

Das System soll, wie bereits ausgeführt wurde, in vorteilhafter Weise mit nur einem Leuchtmittel ausgestattet werden. Um eine Verwendung dieses Leuchtmittels für die DAFE, aber auch für die DWLE zu ermöglichen, ist der Einsatz eines im Sichtbaren breitbandig emittierenden Leuchtmittels vorteilhaft. Die Bereitstellung der geeigneten Strahlung für die Fluoreszenz-Anregung einerseits und die gleichzeitige Bereitstellung der Referenzstrahlung andererseits im Arbeitsmodus der DAFE erfolgt dann durch das Einbringen eines entsprechenden optischen Filters in den Strahlengang des Leuchtmittels. Das Verhältnis der Intensitäten von abgegebener Referenzstrahlung und Fluoreszenz-Anregungstrahlung wird dabei u. a. bestimmt durch das Verhältnis der in den beiden Transmissionsbändern festgelegten spektralen Transmissionsgrade, aber auch durch das Verhältnis der spektralen Breiten der beiden Transmissionsbereiche des optischen Filters.

Nur weil eine Intensität der abgegebenen Referenzstrahlung ausreicht, welche um zwei Größenordnungen geringer ist als die Intensität der Fluoreszenz-Anregungsstrahlung, gelingt es nun, das spektrale Transmissionsband des optischen Filters für die Referenzstrahlung auf ein Minimum an spektraler Breite einzugrenzen, was dann aber durch eine Maximierung des Transmissionsgrades in diesem Band ausgeglichen werden muss. Oder umgekehrt: Je höher der Transmissionsgrad im Band der Referenzstrahlung gewählt wird, um so stärker kann dieses Transmissionsband eingeengt werden. Nur dadurch ist es möglich, das spektrale Band für die Referenzstrahlung so weit einzugrenzen, dass dessen Beitrag für die Farbwiedergabe im Modus der DWLE praktisch keine Rolle mehr spielt. Dabei wird vereinfachend davon ausgegangen, dass die spektrale Intensität des Leuchtmittels über den gesamten sichtbaren Bereich in erster Näherung homogen ist. Bei Abweichungen davon, müssen entsprechende Korrekturen vorgenommen werden. Wird also beispielsweise ein Leuchtmittel gewählt, welches im spektralen Band, welches für die Referenz zuständig ist, eine vergleichsweise starke Emission hat, kann dieses spektrale Band in vorteilhafter Weise noch weiter eingeengt werden.

Theoretisch könnte der spektrale Transmissionsbereich für die Referenzstrahlung auch an einem anderen Ort im Sichtbaren liegen als am kurzwelligen oder am langwelligen Ende des Sichtbaren. Um jedoch bei fester Positionierung des Interferenzfilters im Strahlengang des Leuchtmittels eine nahezu uneingeschränkt gute Farbwiedergabe im Modus der DWLE zu ermöglichen, müsste das Interferenzfilter, welches entsprechend der vorerwähnten Vorgehensweise als Kurz- bzw. als Langpassfilter ausgeführt sein muss, und zwar abhängig von der Wahl der spektralen Lage des Referenzbereiches, dann durch ein optisches Interferenzfilter ersetzt werden, welches in einem dem Wellenlängenband der Referenzstrahlung entsprechenden schmalen Bereich blockt und diesseits und jenseits dieses Wellenlängenbandes eine Transmission von idealerweise nahezu hundert Prozent besitzt. Durch Verkippen des Interferenzfilters könnte dann auch in diesem Fall eine zunehmende Transmission der Referenzstrahlung realisiert werden. Im Sinne einer nahezu uneingeschränkt guten Farbwiedergabe im Modus der DWLE müssten des Weiteren die den Blockbereich begrenzenden spektralen Kanten sehr steil sein. Ein solches optisches Filter ist jedoch mit vertretbarem Aufwand nicht realisierbar, wenn die Auswirkungen für die DWLE vernachlässigbar sein sollen. Daraus ergibt sich die Forderung nach einer Verlagerung desjenigen Spektralbereichs, welchem die Referenzstrahlung entstammt, an das langwellige oder kurzwellige Ende des sichtbaren Spektralbereichs. So kann zur Regulierung der Intensität ein Kantenfilter - abhängig von der Lage des Referenzbereiches entweder ein Kurz- oder Langpassfilter - eingesetzt werden, dessen Transmissionsbereich im Sichtbaren liegt. Dieses Kantenfilter braucht dann erfindungsgemäß nur eine steile spektrale Flanke, welche das Transmissionsband vom Blockband trennt. Solche Kantenfilter wiederum sind relativ einfach und günstig herzustellen.

Weitere vorteilhafte Merkmale der erfindungsgemäßen Vorrichtung ergeben sich aus der Beschreibung eines in der Zeichnung dargestellten Ausführungsbeispiels. In der Zeichnung zeigen:
- Fig. 1: schematisch den Aufbau einer Diagnosevorrichtung, nach der Erfindung,
- Fig. 2: die Optikeinheit der Lichtquelle,
- Fig. 3: eine mögliche Ausführungsform des spektralen Transmissionsverhaltens T des Fluoreszenz-Anregungsfilters über der Wellenlänge W in Nanometer einer ersten Version,
- Fig. 4: die spektrale Hellempfindlichkeit V des menschlichen Auges über der Wellenlänge in Nanometer,
- Fig. 5: das spektrale Transmissionsverhalten T eines Kantenfilters, dessen Spezifikationen auf das Anregungsfilter mit dem in Fig. 3 dargestellten spektralen Transmissionsverhalten T abgestimmt sind, über der Wellenlänge W in Nanometer für unterschiedliche, konstruktiv bedingt mögliche Kippwinkel,
- Fig. 6: ein mögliches spektrales Transmissionsverhalten T des Fluoreszenz-Anregungsfilters über der Wellenlänge W in Nanometer einer zweiten Version und
- Fig. 7: das spektrale Transmissionsverhalten T eines Kantenfilters, dessen Spezifikationen auf das Anregungsfilter mit dem in Fig. 6 dargestellten spektralen Transmissionsverhalten T abgestimmt sind, über der Wellenlänge W in Nanometer für unterschiedliche, konstruktiv bedingt mögliche Kippwinkel.

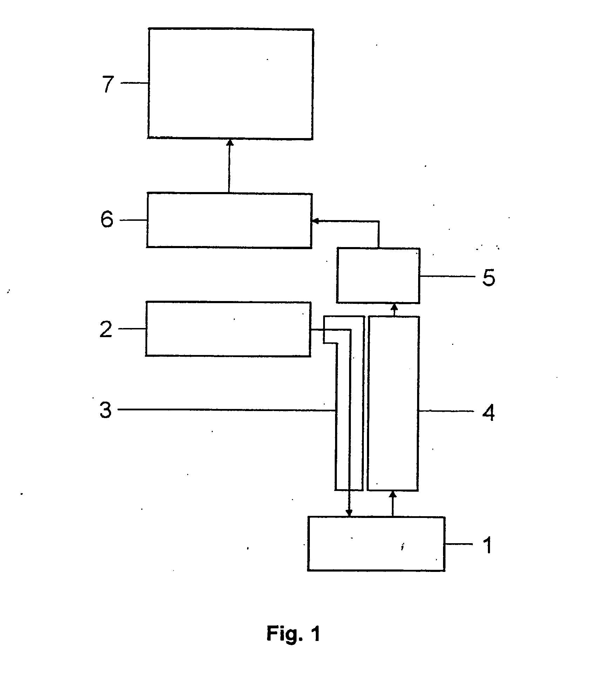

Fig. 1 zeigt schematisch, nämlich in Form eines Blockbildes, den Aufbau der Vorrichtung für die kombinierte diagnostische Weißlicht-Endoskopie DWLE und die diagnostische Autofluoreszenz-Endoskopie DAFE.

Die Vorrichtung zur bildgebenden Diagnose des Gewebes 1 besteht aus einer Lichtquelle 2, welche im Arbeitsmodus der DWLE Weißlicht für die Beleuchtung und im Arbeitsmodus der DAFE Strahlung für die Fluoreszenz-Anregung und Strahlung für die Etablierung einer Farbreferenz über einen Lichtleiter 3 an das Gewebe 1 abgibt. Der Lichtleiter 3 kann aus einer Einzelfaser, einem Faserbündel oder einem Flüssiglichtleiter oder auch aus einer Kombination dieser Elemente bestehen.

Sowohl das bei der DWLE durch remittierte Strahlung erzeugte Bild als auch das bei der DAFE erzeugte Fluoreszenzbild, welchem außerdem remittierte Referenzstrahlung überlagert ist, wird über eine Bildübertragungseinheit 4 einer Bildaufnahmeeinheit 5 zugeführt, wo eine Umwandlung der optischen Signale in elektrische Signale stattfindet. Letztere werden an eine Bildverarbeitungseinheit 6 weitergeleitet, wo eine Aufbereitung der elektrischen Signale stattfindet, um beispielsweise ein Bild auf einem Monitor 7 zu erzeugen. Gleichermaßen ist aber auch die Aufzeichnung mit einem Videorecorder oder einer anderen videotechnischen Einrichtung möglich. Denkbar ist auch die endoskopische Direktbeobachtung, bei welcher die Bildaufnahmeeinheit 5, die Bildverarbeitungseinheit 6 und der Monitor 7 durch das Auge des menschlichen Betrachters ersetzt werden.

Wird für die Bilddarstellung ein Videoendoskop, also ein sog. Chipendoskop, verwendet, bei welchem der Videochip im Endoskop selbst untergebracht ist, dann handelt es sich bei der Bildübertragungseinheit 4 im Wesentlichen um ein Objektiv und bei der Bildaufnahmeeinheit 5 um das Sensorsystem des Videoendoskops. Die Bildverarbeitungseinheit 6 stellt den zugehörigen Controller dar. Dabei handelt es sich sowohl beim Chipendoskop als auch beim Controller um Komponenten, die uneingeschränkt für die DWLE verwendet werden können.

Findet hingegen eine Kamera Verwendung, dann besteht die Bildübertragungseinheit 4 aus einem Endoskop-Objektiv, einem sich daran anschließenden Bildfaserbündel oder einem Linsenoder Stablinsensystem, welche beide Teil eines Endoskops sind, aus einem Endoskop-Okular und evtl. einem Kamera-Objektiv. Diese Komponenten übertragen das Bild vom Gewebe auf die Bildaufnahmeeinheit 5. Letztere wird gebildet vom Sensorsystem eines Kamerakopfes. Bei der Bildverarbeitungseinheit 6 handelt es sich um den Kameratreiber. Auch bei der Kamera handelt es sich um ein Gerät, welches uneingeschränkt für die DWLE verwendet werden kann. Es kann sich sogar um eine konventionelle medizinische Kamera handeln.

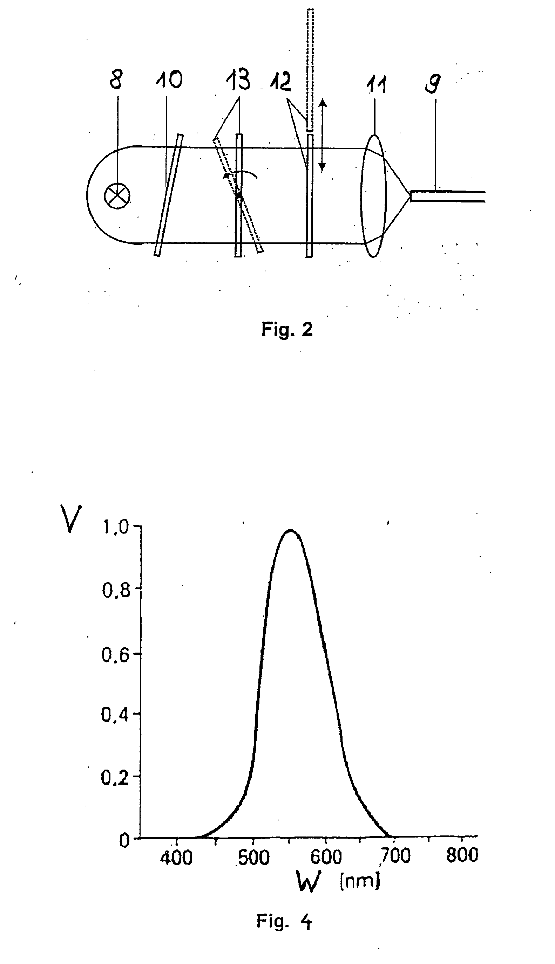

Fig. 2 zeigt die Optikeinheit der Lichtquelle 2 aus Fig. 1. Bei dem in dieser Lichtquelle verwendeten Leuchtmittel 8 handelt es sich um eine inkohärente, breitbandig im sichtbaren Spektralbereich emittierende Lampe. Wird bei dem idealerweise weißes Licht emittierenden Leuchtmittel eine Kurzbogenlampe verwendet, beispielsweise eine Xenonlampe, eine Mischgaslampe mit entsprechender Gasmischung oder eine Lampe mit Quecksilberanteilen, gelingt die Strahlungseinkopplung in den Lichtleiter 9, welcher dem Lichtleiter 3 in Fig. 1 entspricht, besonders gut. Alternativ kann als Leuchtmittel auch eine Halogenlampe verwendet werden.

Im Ausführungsbeispiel der Fig. 2 findet eine Parabolspiegellampe Verwendung, welche ein paralleles Strahlenbündel abgibt. Das sich fest im Strahlengang befindliche Filter 10 blockiert die UV- und die IR-Strahlungsanteile des Leuchtmittels 8. Die Linse 11 fokussiert das parallele Strahlenbündel des Leuchtmittels 8. Im Brennpunkt der Linse 11 befindet sich der Lichtleiter 9, in welchen die gefilterte Strahlung des Leuchtmittels eingekoppelt wird.

Beim Umschalten in den Arbeitsmodus der DAFE wird das Fluoreszenz-Anregungsfilter 12 in den parallelen Strahlengang des Leuchtmittels 8 eingeschwenkt. Dies geschieht beispielsweise über das Betätigen eines Fußschalters oder über einen Tastendruck an der Bedienfront der Lichtquelle. Beide Bedienelemente sind mit einer Steuereinheit verbunden, die den Filterschwenkmechanismus kontrolliert und die wiederum in der Lichtquelle untergebracht sein kann. Diese den Schwenkvorgang von Filter 12 betreffenden Komponenten sind in Fig. 2 nicht abgebildet. Beim Umschalten in den Arbeitsmodus der DWLE wird das Fluoreszenzanregungsfilter 12 aus dem Strahlengang ausgeschwenkt. Dies geschieht in der gleichen Weise wie das Einschwenken.

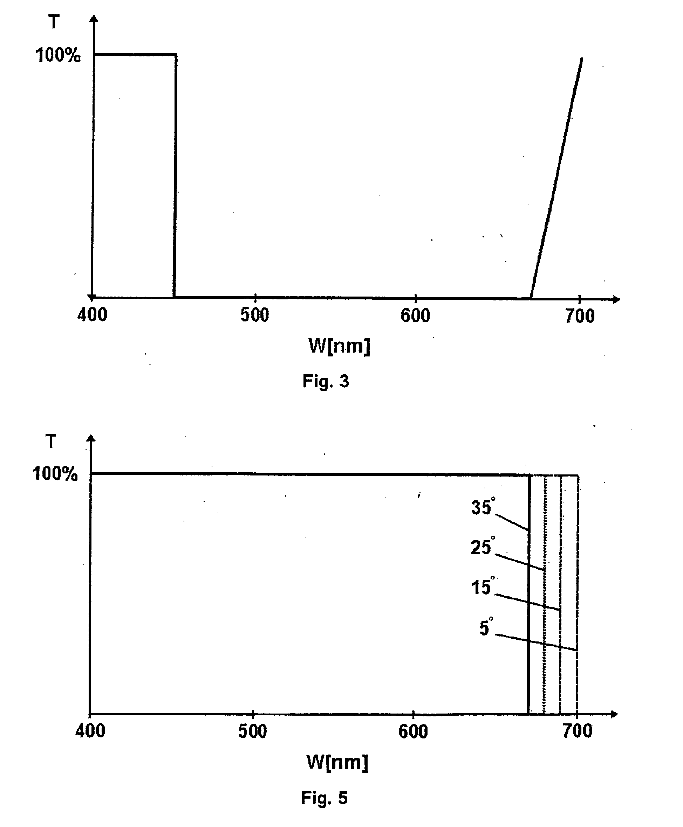

Fig. 3 zeigt schematisch eine mögliche Ausführungsform einer ersten Version des spektralen Transmissionsverhaltens des Fluoreszenz-Anregungsfilters 12. Ein erster Transmissionsbereich des Filters liegt im Violetten und Blauen und dient der Fluoreszenz-Anregung. Der spektrale Transmissionsgrad in diesem Bereich geht idealerweise gegen hundert Prozent, um eine bestmögliche Fluoreszenz-Anregung im Sinne eines hellen Fluoreszenzbildes zu erzeugen.

Diese erste Version ist dadurch gekennzeichnet, dass die zu bildende Farbreferenz dem roten Spektralbereich entstammt. Entsprechend den vorausgegangenen Überlegungen und im Sinne der Erfindung ist dann der für die Farbreferenz gewählte, zweite Transmissionsbereich des Filters vergleichsweise schmal und beginnt am Rande des sichtbaren Spektrums im langwelligen Roten, so dass diese spektralen Anteile für die Farbwiedergabe beim konventionellen Weißlichtbild keine merkliche Rolle spielen. Dieser für die Bildung der Farbreferenz zuständige zweite Transmissionsbereich des Filters beginnt bei der vorgestellten möglichen Ausführungsform bei etwa 665nm, liegt bei 670nm im Prozentbereich und steigt idealerweise mit zunehmender Wellenlänge stark an, um die gleichzeitig abnehmende spektrale Hellempfindlichkeit V des Auges (siehe Fig. 4) - und daran angepasst die spektrale Detektion einer konventionellen Bildaufnahmeeinheit - zu kompensieren. Unterhalb einer Wellenlänge von 665nm bis zum ersten Transmissionsband des Filters ist der spektrale Transmissionsgrad von Filter 12 idealerweise bei null Prozent.

Außerdem befindet sich ein Kantenfilter 13 im Strahlengang der Lichtquelle. Dieses Filter besteht aus einem optisch transparenten, dünnen Plättchen, auf welches dielektrische Schichten aufgebracht sind, welche das Transmissionsverhalten des Filters bestimmen. Dieses Filter bleibt im Sinne eines konstruktiv einfachen Aufbaus und damit im Sinne der Erfindung immer im Strahlengang der Lichtquelle, d. h., sowohl bei der DAFE als auch bei der DWLE. Das Filter ist derart im Strahlengang der Lichtquelle angebracht, dass es in einem konstruktiv vorgegebenen, genau definiertem Winkelbereich gegen das parallele Strahlenbündel verkippt werden kann. Die sich daraus ergebenden Grenzen der möglichen spektralen Kantenlagen des Filters sind dadurch genau festgelegt.

Wie schon an anderer Stelle erläutert wurde, sind die Spezifikationen von Kantenfilter 13 und Fluoreszenz-Anregungsfilter 12 so aufeinander abgestimmt, dass sich die spektrale Kante des Kantenfilters 13 im konstruktiv möglichen Kippbereich ausschließlich über denjenigen schmalen Transmissionsbereich des Fluoreszenz-Anregungsfilters 12 bewegt, welcher die Strahlung für das Referenzlicht bereitstellt, und zwar in der Art, dass bei der dargestellten möglichen Ausführungsform der ersten Version die Lage der spektralen Kante des Filters bei keiner Wellenlänge sein kann, die kleiner als 665 nm ist. Dadurch ist gewährleistet, dass einerseits die Intensität der zusätzlich bereitgestellten Rotreferenz über einen weiten Bereich dosiert werden kann und sogar der gesamte Anteil an zusätzlichem, zur Bildung einer Farbreferenz bereitgestelltem Rotlicht eliminiert werden kann und andererseits die Farbwiedergabe bei der DWLE auch bei maximal eingestelltem Kippwinkel des Kantenfilters 13 im Strahlengang des Leuchtmittels nicht merklich beeinträchtigt wird.

Fig. 5 zeigt skizzenhaft das spektrale Transmissionsverhalten T einer möglichen Ausführungsform des Kantenfilters 13 gemäß Fig. 2, welches auf das Fluoreszenz-Anregungsfilter 12 nach Fig. 2 mit dem in Fig. 3 dargestellten spektralen Transmissionsverhalten abgestimmt ist, für unterschiedliche, konstruktiv mögliche Kippwinkel. Bei einem maximal möglichen Kippwinkel in der Größenordnung von 35 Grad ist gewährleistet, dass nahezu das gesamte Referenzlicht licht eliminiert ist und dennoch die Farbqualität bei der DWLE keine wahrnehmbaren Einbußen erfährt. Mit abnehmendem Kippwinkel verlagert sich die spektrale Kante zunehmend in den langwelligen Spektralbereich, und der Anteil an rotem Referenzlicht, mit welchem das Gewebe beaufschlagt wird und welches dort remittiert wird und den Detektor erreicht, nimmt zu. Gleichzeitig wird der Einfluss des Filters auf die die Farbwiedergabe im Modus der DWLE unkritischer. Die erfindungsgemäße Vorgehensweise erlaubt es damit, die im Sinne einer optimalen Gewebedifferenzierung kritische Dosierung und Einstellung der Intensität der Referenzstrahlung während einer Patientenuntersuchung nur einmal vornehmen zu müssen und dennoch in einfachster Form sowohl hinsichtlich des Systemaufbaus aber auch im Hinblick auf die Anwenderfreundlichkeit zwischen der DWLE und der DAFE zu wechseln, ohne irgendwelche Beeinträchtigungen, vor allen Dingen bezüglich der Bildqualität, erfahren zu müssen. Es ist lediglich ein zusätzliches, verkippbares einfach herzustellendes steilflankiges Kantenfilter im Strahlengang des Leuchtmittels erforderlich, wobei es sich bei dieser ersten Version um ein Kurzpassfilter handeln muss, um eine Transmission der Strahlung aus dem sichtbaren Spektralbereich zu ermöglichen. Bei entsprechender Spezifikation kann übrigens das Kanten- bzw. Kippfilter 3 das Filter 10 ersetzen.

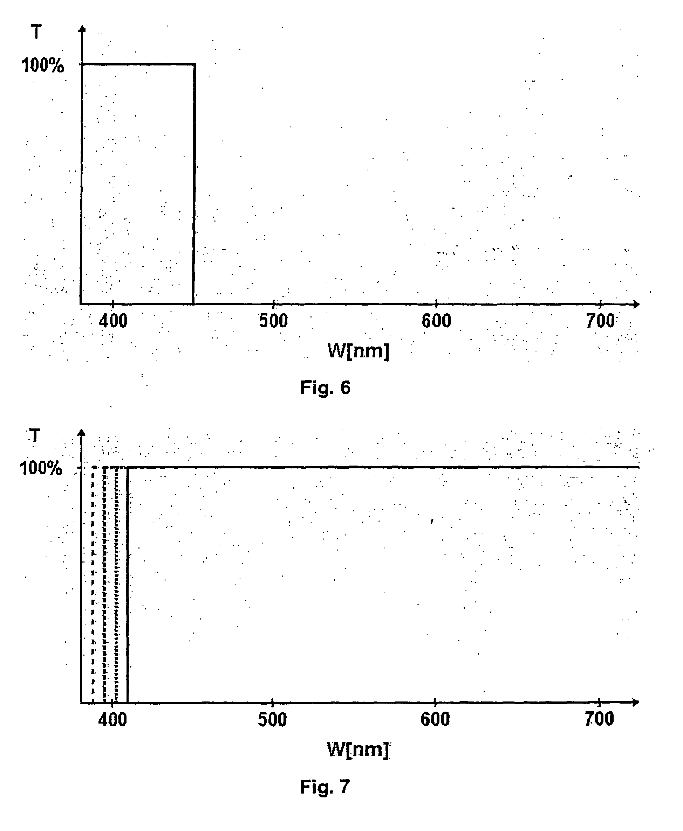

Einen möglichen Verlauf des spektralen Transmissionsverhaltens T des Fluoreszenz-Anregungsfilters einer zweiten Version zeigt schematisch Fig. 6. Diese zweite Version ist dadurch gekennzeichnet, dass der spektrale Transmissionsbereich, welcher für die Farbreferenz verantwortlich ist, ein schmales Band ist, welches am kurzwelligen Ende des sichtbaren Spektralbereichs liegt und welches mit dem spektralen Band für die Fluoreszenz-Anregung verschmilzt.

Fig. 7 zeigt schematisch das spektrale Transmissionsverhalten T eines Kantenfilters, dessen Spezifikationen auf das Anregungsfilter mit dem in Fig. 6 dargestellten spektralen Transmissionsverlauf abgestimmt sind, für unterschiedliche, konstruktiv bedingt mögliche Kippwinkel. Bei dieser zweiten Version muss im Filterdesign berücksichtigt werden, dass eine Erhöhung der Intensität der Referenzstrahlung eine verstärkte Fluoreszenz-Anregung bedeutet und damit mit einem Ansteigen an Fluoreszenz einhergeht. Außerdem muss darauf geachtet werden, dass das Anregungslicht-Blockfilter im Bildpfad so spezifiziert ist, dass es zwar einerseits das Fluoreszenz-Anregungslicht blockt, aber andererseits Strahlung aus dem kurzwelligen schmalen spektralen Bereich, welcher für die Referenz zuständig ist, transmittiert.

## Patentansprüche

1. Vorrichtung zur bildgebenden Diagnose von Gewebe (1) unter wahlweiser Anwendung von zwei Diagnosemethoden, nämlich einem Arbeitsmodus zur diagnostischen Weißlicht-Endoskopie DWLE und einem Arbeitsmodus zur diagnostischen Autofluoreszenz-Endoskopie DAFE, mit einer Lichtquelle (2), deren Licht über einen Lichtleiter (3) zum Gewebe (1) geleitet wird, mit einer Bildübertragungseinheit (4), die zur endoskopischen Direktbeobachtung ausgebildet ist oder über eine Bildaufnahmeeinheit (5) mit daran angeschlossener Bildverarbeitungseinheit (6) mit einem Monitor (7) verbunden ist, und mit einem in den Strahlengang der Lichtquelle (2) bewegbaren Fluoreszenzanregungsfilter (12), **dadurch gekennzeichnet, dass** das Fluoreszenzanregungsfilter (12) neben einem Transmissionsbereich für eine Fluoreszenzanregungsstrahlung einen weiteren am kurzwelligen oder am langwelligen Ende des sichtbaren Spektralbereichs liegenden Transmissionsbereich für eine Referenzstrahlung aufweist und dass in dem Strahlengang der Lichtquelle (2) ein zur Intensitätsänderung der Referenzstrahlung ausgebildetes verstellbares Kantenfilter (13) angeordnet ist.

2. Vorrichtung nach Anspruch 1, **dadurch gekennzeichnet, dass** die Verstellung der Intensität der Referenzstrahlung über das Verkippen eines Kantenfilters (13) erfolgt, dessen Filterwirkung auf dielektrischen Schichten basiert und dessen Transmissionsbereich den gesamten sichtbaren Spektralbereich zumindest teilweise einschließt.

3. Vorrichtung nach Anspruch 2, **dadurch gekennzeichnet, dass** der Kippwinkelbereich des Kantenfilters (13) mechanisch-konstruktiv so eingeschränkt ist, dass sich die spektrale Kante des Filters (13) nur über den Spektralbereich bewegen kann, welchem die Referenzstrahlung entstammt.

4. Vorrichtung nach einem der Ansprüche 2 und 3, **dadurch gekennzeichnet, dass** das Kantenfilter (13) auch beim Wechsel vom Arbeitsmodus der DAFE in den Arbeitsmodus der DWLE in seiner bei der DAFE eingestellten und optimierten Position im Strahlengang des Lichtquelle (2) bleibt.

5. Vorrichtung nach einem der Ansprüche 1 bis 4, **dadurch gekennzeichnet, dass** das spektrale Band, welchem die Referenzstrahlung entstammt, im Bereich von 650 bis 720 nm oder im Bereich von 390 bis 440 nm liegt.

6. Vorrichtung nach einem der Ansprüche 1 bis 5, **dadurch gekennzeichnet, dass** die Bildübertragungseinheit (4) aus dem Objektiv eines Chipendoskops, die Bildaufnahmeeinheit (5) aus dem Sensorsystem eines Chipendoskops und die Bildverarbeitungseinheit (6) aus dem zugehörigen Controller besteht.

7. Vorrichtung nach einem der Ansprüche 1 bis 5, **dadurch gekennzeichnet, dass** die Bildübertragungseinheit (4) aus dem Objektiv, aus dem Linsensystem/ Bildfaserbündel und dem Okular eines Endoskops sowie aus dem Kameraobjektiv besteht und dass die Bildaufnahmeeinheit (5) aus dem Sensorsystem einer Kamera und die Bildverarbeitungseinheit (6) aus dem zugehörigen Treiber besteht.

8. Vorrichtung nach Anspruch 1, **dadurch gekennzeichnet, dass** die Lichtquelle (2) aus einem inkohärenten, spektral breitbandig emittierenden Leuchtmittel besteht.

## Claims

1. A device for the imaging diagnosis of tissue (1), with the selective application of two diagnosis methods, specifically an operating mode for diagnostic white light endoscopy DWLE and an operating mode for diagnostic auto-fluorescence endoscopy DAFE, with a light source (2), whose light is led via a light guide (3) to the tissue (1), with a picture transmission unit (4) which is designed for endoscopic direct observation or is connected to a monitor (7) via a picture recording unit (5) with a picture processing unit (6) connected thereto, and with a fluorescence excitation filter (12) which is movable into the beam path of the light source (2), **characterised in that** the fluorescence excitation filter (12) apart from a transmission region for a fluorescence excitation radiation, comprises a further transmission region for reference radiation, which lies at the short-wave or long-wave end of the visible spectral region, and that an adjustable cut-off filter (13) which is designed for the intensity change of the reference radiation, is arranged in the beam path of the light source (2).

2. A device according to claim 1, **characterised in that** the adjustment of the intensity of the reference radiation is effected via the tilting of a cut-off filter (13), whose filter effect is based on dielectric layers and those transmission region at least partly includes the whole visible spectral region.

3. A device according to claim 2, **characterised in that** the tilt angle region of the cut-off filter (13) is limited by way of mechanical design, such that the spectral edge of the filter (13) may only move over the spectral region, from which the reference radiation originates.

4. A device according to one of the claims 2 and 3, **characterised in that** the cut-off filter (13), also with the change from the operating mode of DAFE into the operating mode of DWLE, remains in its position in the beam path of the light source (2), said position set and optimised with DAFE.

5. A device according to one of the claims 1 to 4, **characterised in that** the spectral band from which the reference radiation originates, lies in the region of 650 to 720 nm or in the region of 390 to 440 nm.

6. A device according to one of the claims 1 to 5, **characterised in that** the picture transmission unit (4) consists of the objective of a chip endoscope, the picture recording unit (5) consists of the sensor system of a chip endoscope, and the picture processing unit (6) consists of the associated controller.

7. A device according to one of the claims 1 to 5, **characterised in that** the picture transmission unit (4) consists of the objective, of the lens system/picture fiber bundle and of the eyepiece of an endoscope as well as of the camera objective, and that the picture recording unit (5) consists of the sensor system of a camera, and the picture processing unit (6) consists of the associated driver.

8. A device according to claim 1, **characterised in that** the light source (2) consists of an incoherent, light means emitting in a spectrally broad-band manner.

## Revendications

1. Dispositif de diagnostic de tissu par formation d'image (1) en utilisant au choix deux méthodes de diagnostic, à savoir un mode de travail permettant une endoscopie diagnostique en lumière blanche (DWLE) et un mode de travail permettant une endoscopie diagnostique par autofluorescence (DAFE), comportant une source lumineuse (2) dont la lumière est dirigée vers le tissu (1) via une fibre optique (3), comportant une unité de transmission des images (4) qui est conçue pour une visualisation endoscopique directe ou est reliée à un écran (7) via une unité d'enregistrement des images (5) avec unité de traitement des images (6) qui y est raccordée, et comportant un filtre d'excitation pour la fluorescence (12) mobile dans la trajectoire des rayons de la source lumineuse (2), **caractérisé en ce que** le filtre d'excitation pour la fluorescence (12), outre une plage de transmission pour un rayon d'excitation de la fluorescence, présente une autre plage de transmission pour un rayon de référence située à l'extrémité ondes courtes ou grandes ondes de la plage spectrale visible, et **en ce que** dans la trajectoire des rayons de la source lumineuse (2) est disposé un filtre à arêtes réglable (13) conçu pour modifier l'intensité du rayon de référence.

2. Dispositif selon la revendication 1, **caractérisé en ce que** le réglage de l'intensité du rayon de référence se fait par le basculement du filtre à arêtes (13) dont l'effet de filtre repose sur des couches diélectriques et dont la plage de transmission englobe au moins partiellement l'ensemble de la plage spectrale visible.

3. Dispositif selon la revendication 2, **caractérisé en ce que** la plage d'angle de basculement du filtre à arêtes (13) est limitée de par la conception mécanique de façon que l'arête spectrale du filtre (13) ne peut se déplacer que dans la plage spectrale d'où provient le rayonnement de référence.

4. Dispositif selon l'une des revendications 2 et 3, **caractérisé en ce que** le filtre à arêtes (13) reste dans la trajectoire des rayons de la source lumineuse (2), même en cas de changement du mode de travail DAFE en mode de travail DWLE, dans sa position réglée en mode DAFE et optimisée.

5. Dispositif selon l'une des revendications 1 à 4, **caractérisé en ce que** la bande spectrale d'où provient le rayonnement de référence est comprise dans la plage de 650 à 720 nm ou dans la plage de 390 à 440 nm.

6. Dispositif selon l'une des revendications 1 à 5, **caractérisé en ce que** l'unité de transmission des images (4) se compose de l'objectif d'un endoscope à puce, l'unité d'enregistrement des images (5) du système de capteur d'un endoscope à puce et l'unité de traitement des images (6) du contrôleur associé.

7. Dispositif selon l'une des revendications 1 à 5, **caractérisé en ce que** l'unité de traitement des images (4) se compose de l'objectif, du système à lentilles/du faisceau de fibres d'image et de l'oculaire d'un endoscope, ainsi que de l'objectif de caméra, et **en ce que** l'unité d'enregistrement des images (5) se compose du système de capteur d'une caméra, et l'unité de traitement des images (6) du pilote associé.

8. Dispositif selon la revendication 1, **caractérisé en ce que** la source lumineuse (2) se compose d'un moyen d'éclairage à émission incohérente en large bande spectrale.
